# EUROPEAN PATENT APPLICATION

(11) **EP 4 197 323 A1**
(43) Date of publication of application: **21.06.2023**
(21) Application number: 21856056.3
(22) Date of filing: 24.06.2021
(51) Int. Cl.: A01K 67/027

(54) **NOVEL METHOD FOR PREPARING ANIMAL MODEL OF CEREBROVASCULAR DISEASE AND METHOD FOR PRODUCING ANIMAL HAVING SMALL INDIVIDUAL DIFFERENCE IN SUSCEPTIBILITY TO CEREBROVASCULAR DISEASE ONSET BY USING ANIMAL MODEL FOR CEREBROVASCULAR DISEASE PREPARED THEREBY**

(30) Priority: 13.08.2020 KR 20200101553
(71) Applicant: Dongguk University Industry-Academic Cooperation Foundation, Seoul 04620 (KR)
(72) Inventor: KIM, Dong Eog, Seoul 06509 (KR)
(74) Representative: Cabinet Nony
(86) International application number: PCT/KR2021/007949
(87) International publication number: WO 2022/035049

(57) **Abstract**

The present invention relates to a method for producing animal models of cerebrovascular disease, comprising ligation of the common carotid artery (CCA) and administration of a nitric oxide (NO) inhibitor, animal models of cerebrovascular disease produced thereby, and a method for producing animals having small individual differences in susceptibility to the onset of cerebrovascular disease by using the same. The method for producing animal models of cerebrovascular disease according to the present invention can overcome the conventional problems of complexity and low yield. Particularly, the production method of the present invention can produce animal models of cerebrovascular disease with a high yield by a simple method. Moreover, the animal models produced by the production method may be used in studies to verify the effectiveness and safety of substances for the diagnosis, prevention or treatment of cerebrovascular disease or methods for treatment of cerebrovascular disease, and make it possible to study genes that cause differences in the level of cerebrovascular disease between the animal models. In addition, it is possible to provide offspring animals having uniform susceptibility to the onset of cerebrovascular disease by mating the animal models having similar susceptibilities to the onset of cerebrovascular disease according to the present invention.

## Description

### Technical Field

The present invention relates to a novel method for producing an animal model of cerebrovascular disease and a method for producing animals having small inter-individual differences in susceptibility to cerebrovascular diseases by using the new animal model.

### Background Art

Cerebral neurovascular diseases, including cerebral infarction and cerebrovascular dementia, are diseases with high incidence, prevalence, mortality, and disability rates. Therefore, studies using various animal models have been widely conducted worldwide to develop technology for diagnosing and treating cerebral neurovascular diseases.

Cerebral infarction is caused by blockage of cerebral blood vessels and the frequency thereof is about 3 to 4 times higher than that of cerebral hemorrhage caused by rupture of cerebral blood vessels. The most widely used method for producing an animal model of cerebral infarction is either the Longa method, in which a small opening is made on the carotid artery, a nylon suture is inserted into the opening while minimizing bleeding, and then the suture is carefully pushed in while taking care not to let the animal die due to blood vessel perforation or the like, thereby blocking the middle cerebral artery, or a modification of the Longa method. This method involves very invasive and technically demaning microvascular surgery, and is a method that is difficult to learn and is highly prone to experimental failures even after a long period of learning. Also, it is difficult to efficiently conduct large artery ischemic stroke research using this method, because one researcher can usually make about 3 to 4 animals per day due to the demanding work load. In addition, it is often difficult to judge whether the cause of death is due to cerebral infarction itself or invasive surgical procedure-related stress. Since there is no animal model of large arterial cerebral artery infarction that could be induced without these problems, there is an urgen need for developing a new and easier model of large ischemic infarction.

In addition, there are big inter-strain or inter-individual differences in susceptibility to cerebrovascular diseases due to variabilities in the neurovascular anatomy and pathophysiology. For example, in the case of C57BL/6 mice, which are the most widely used worldwide in stroke experiments, the presence or absence or degree of development of the posterior communicating artery collaterals, which are used as a bypass when cerebral vessels such as the middle cerebral artery are blocked and blood flow to the brain is blocked, is highly variable. Thus, variabilities in the size of cerebral infarction inevitably follows even when animal experiments are conducted in the same manner. If the sizes of cerebral infarction in untreated control animals are not uniform, it is inevitably difficult to secure the reliability of the experiments to confirm the effect of treatment. Currently, it is usually difficult to predict individual susceptibility to cerebrovascular diseases before the production of a cerebral infarction model. For example, it is difficult to know the posterior communicating artery and pial collateral status in vivo. It will be useful if researchers have mouse strains with a consistent level of vulnerability to cerebrovascular diseases. In order to overcome these problems, sub- strains with a low variability in the anatomic and pathophysiologic susceptibility to cerebrovascular diseases, such as the collateral stataus and neurovascular resistance to ischemia, are required. However, there is no selective breeding method for producing such animals, and thus it is necessary to develop this method. To date, C57Bl/6 mice have been frequently used in cerebral infarction experiments, and numerous genetically modified mice have been established using the C57Bl/6 mice strain, however it is not easy to perform a comparative assessment of the susceptibility to cerebrovascular diseases based on the posterior communicating artery and pial collateral status in vivo. In addition, collateral status is not the only factor that affects the variability to cerebrovascular diseases, such as the size of cerebral infarction and post-stroke neurological dysfunction. In order to overcome these problems, a better comparative tool to assess the susceptibility to cerebrovascular diases is required. It is notable that the aforementioned unmet needs are related to each other, so there should be a method to simultaneously achieve all objectives.

### DISCLOSURE

### Technical Problem

An object of one aspect of the present invention is to provide a method for producing animal models of cerebrovascular disease, the method comprising ligation of a common carotid artery (CCA) and administration of a nitric oxide (NO) inhibitor.

An aspect of another aspect of the present invention is to provide animal models of cerebrovascular disease produced by the above method.

An object of still another aspect of the present invention is to provide a method for producing animal models having uniform susceptibility to the onset of cerebrovascular disease, the method comprising a step of mating animal models having similar susceptibilities to the onset of cerebrovascular disease, among the animal models of cerebrovascular disease.

An object of yet another aspect of the present invention is to provide animal models having uniform susceptibility to the onset of cerebrovascular disease, produced by the above method.

### Technical Solution

One aspect of the present invention provides a method for producing animal models of cerebrovascular disease, the method comprising ligation of a common carotid artery (CCA) and administration of a nitric oxide (NO) inhibitor.

In one embodiment of the present invention, the ligation of the common carotid artery (CCA) may be permanent ligation.

In one embodiment of the present invention, the nitric oxide (NO) inhibitor may be any one or more of NG-nitro-L-arginine methyl ester (L-NAME), NG-monoethyl-L-arginine (L-MMA), N-iminoethyl-L-ornithine (L-NIO), L-monomethyl-L-arginine (L-NNMA), L-NG-methylarginine (L-NMA), Nw-nitro-L-arginine (L-NA), and aminoguanidine.

In another embodiment of the present invention, the administration of the nitric oxide (NO) inhibitor may be by any one route selected from among oral, parenteral, intravascular, intradermal, transdermal, intramuscular, intraperitoneal, intravenous, subcutaneous, and intranasal routes.

In another embodiment of the present invention, the administration of the nitric oxide (NO) inhibitor may be performed before or after the ligation of the common carotid artery.

In one embodiment of the present invention, the cerebrovascular disease may be at least one selected from the group consisting of palsy, stroke, cerebral hemorrhage, cerebral infarction, Alzheimer's disease, and vascular dementia.

Another aspect of the present invention provides animal models of cerebrovascular disease produced by the above method.

In one embodiment of the present invention, the cerebrovascular disease may be acute cerebral infarction.

Still another aspect of the present invention provides a method for producing offspring animals having uniform susceptibility to the onset of cerebrovascular disease, the method comprising a step of mating animal models having similar susceptibilities to the onset of cerebrovascular disease among the above-described animal models of cerebrovascular disease.

Yet another aspect of the present invention provides offspring animas having uniform susceptibility to the onset of cerebrovascular disease, produced by the above method.

### Advantageous Effects

The method for producing animal models of cerebrovascular disease according to the present invention does not require complicated steps such as conventional invasive microsurgery, forced blood pressure reduction, or oxygen supply cutoff, requires a 5 to 10-fold shorter time for model production, and thus may overcome the low-yield problem. Specifically, the production method of the present invention is capable of producing animal models of cerebrovascular disease with high yield by a simple method. Furthermore, the animal models produced by the above production method may be used in studies to verify the effectiveness and safety of substances for the diagnosis, prevention or treatment of cerebrovascular disease or methods for treatment of cerebrovascular disease, and make it possible to study genes that cause differences in the level of cerebrovascular disease between animal models. In addition, the onset of cerebrovascular disease in the animal models may be accurately detected non-invasively through brain imaging equipment such as laser speckle flowmetry without expensive equipment such as MRI, which is necessary to confirm the onset of cerebrovascular disease in conventional animal models.

In addition, it is possible to provide offspring animals having uniform susceptibility to the onset of cerebrovascular disease by mating animal models having similar susceptibilities to the onset of cerebrovascular disease among the above-described animal models of cerebrovascular disease.

### Brief Description of Drawings

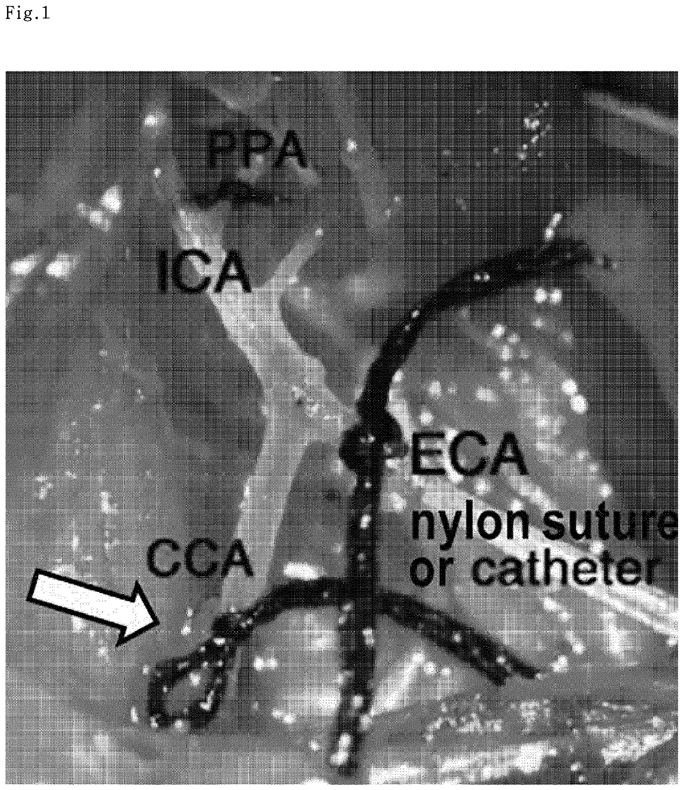

FIG. 1 is a photograph showing an operation for producing a conventional animal model of cerebrovascular disease.

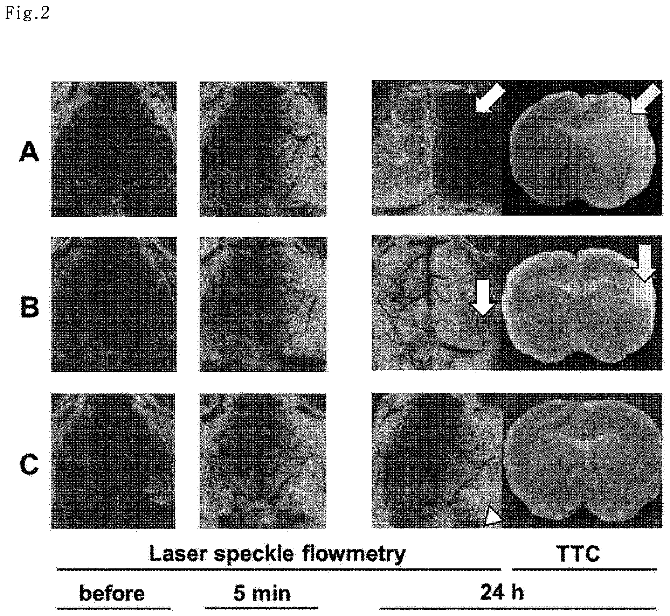

FIG. 2 depicts laser speckle flowmetry images of animal models produced by the method of producing animal models of cerebrovascular disease according to the present invention and photographs showing the results of performing brain tissue staining with 2,3,5-triphenyl tetrazolium chloride (TTC) .

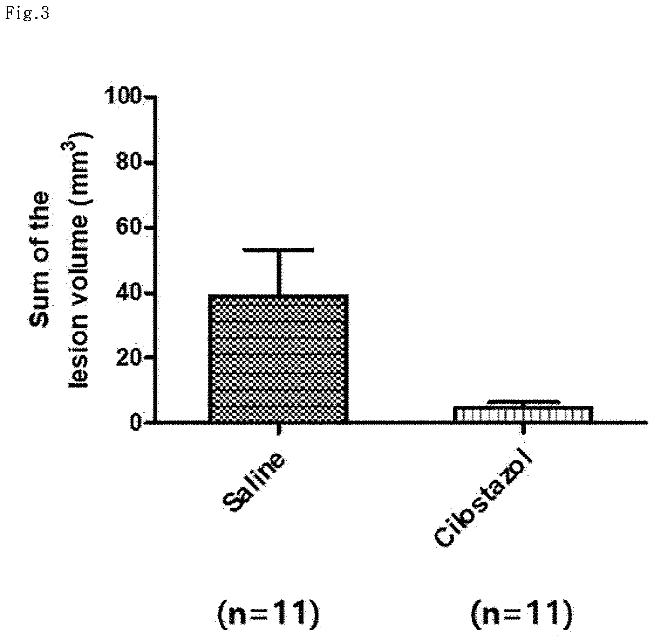

FIG. 3 is a graph showing that the animal models of the present invention may be used as animal models. Specifically, FIG. 3 shows that the size of cerebral infarction induced in the cerebral infarction animal models of the present invention is reduced by administration of cilostazol that is a therapeutic agent for cerebral infarction.

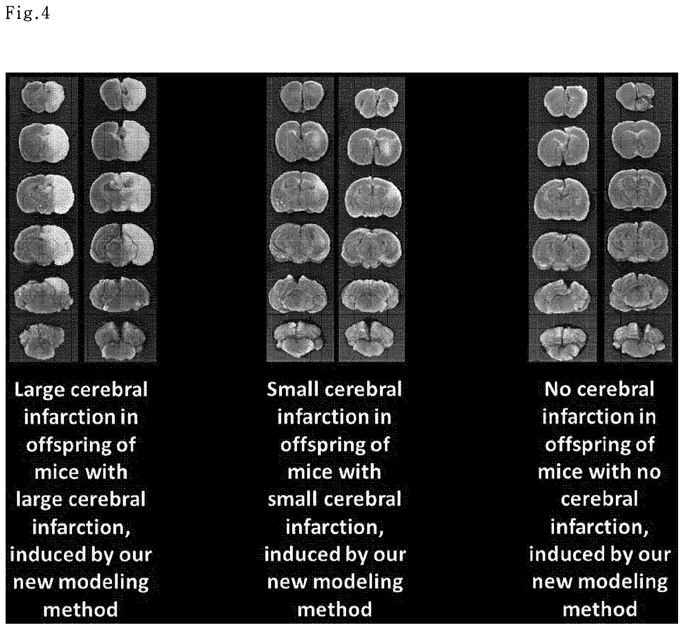

FIG. 4 is a photograph showing that the gene determining the level of cerebrovascular disease in the animal models according to the present invention may be inherited to the next generation, thus making it possible to provide offspring animals having uniform susceptibility to cerebrovascular disease. Specifically, FIG. 4 is a photograph showing that, when the method for producing animal models of cerebrovascular disease according to the present invention is applied to offspring animal models obtained by mating animal models having similar susceptibilities to cerebrovascular disease among the animal models of the present invention, the offspring animal models have cerebrovascular disease susceptibility equivalent to that of their parents.

### Best Mode

One aspect of the present invention provides a method for producing animal models of cerebrovascular disease, the method comprising ligation of a common carotid artery (CCA) and administration of a nitric oxide (NO) inhibitor.

The method for producing animal models of cerebrovascular disease according to the present invention comprises ligation of a common carotid artery (CCA). Acute cerebral infarction does not occur even when blood circulation flowing into the brain is partially restricted through such common carotid artery ligation, but acute cerebral infarction may be induced by administering a nitric oxide inhibitor.

In a conventional art, an animal model of cerebrovascular disease has been produced by partially incising the carotid artery to make an opening and inserting a nylon suture into the opening while minimizing bleeding, thus blocking the middle cerebral artery, but there is a problem in that the difficulty is high and the yield is not good. However, in the present invention, there is no surgical procedure other than ligation of the common carotid artery, and thus there are effects in that the difficulty is not high and the yield is increased.

As used herein, the term "common carotid artery (CCA)" refers to a main blood vessel that supplies blood from the aorta in the chest through the neck to the brain, and is also called the carotid artery. It is a pair of left and right blood vessels that run anteriorly and supply blood to most of the cerebrum except for the back of the brain.

As used herein, the term "ligation" refers to stopping blood circulation in a certain part of a blood vessel or tissue. The ligation may be performed by tying a knot or using a device such as forceps. Specifically, examples of a method for tying a knot include surgical knot, male knot tying, female knot tying, and local ligation that is performed using forceps. Examples of sutures used for ligation through knot tying include, but are not limited to, non-absorbent silk sutures, nylon sutures, dacron sutures, and absorbent winding sutures, and the like.

In one embodiment of the present invention, the ligation of the common carotid artery (CCA) may be permanent ligation. That is, the ligation of the common carotid artery (CCA) of the present invention may be permanent ligation achieved by tying a knot to the common carotid artery (CCA), without being limited thereto.

The method for producing animal models of cerebrovascular disease according to the present invention comprises administration of a nitric oxide (NO) inhibitor. Only administration of the nitric oxide inhibitor does not cause cerebral infarction, but if blood flow to the brain is restricted through ligation of the common carotid artery while contracting cerebral blood vessels or obstructing cerebrovascular dilatation through administration of the nitric oxide (NO) inhibitors, cerebrovascular disease is induced.

The method for producing animal models of cerebrovascular disease according to the present invention may produce animal models of cerebrovascular disease even by only a simple operation comprising the ligation of the common carotid artery (CCA) and the administration of the nitric oxide (NO) inhibitor, and thus may overcome the conventional problem that the yield of animal models of cerebrovascular disease is low due to complexity and high difficulty.

The "nitric oxide (NO)" that is used in the present invention is a substance that is formed from arginine within cells and involved in various physiological activities such as immune action, vasodilation and signaling in the body. Specifically, in mammalian cells, nitric oxide is produced as arginine is oxidized to nitric oxide and citrulline by nitric oxide synthase (NOS). This nitric oxide is known to dilate blood vessels, thereby facilitating blood supply and lowering blood pressure.

In one embodiment of the present invention, the nitric oxide (NO) inhibitor may be a substance that inhibits the production of nitric oxide and/or a substance that removes the produced nitric oxide. The substance that inhibits the production of nitric oxide may be any one or more of NG-nitro-L-arginine methyl ester (L-NAME), NG-monoethyl-L-arginine (L-MMA), N-iminoethyl-L-ornithine (L-NIO), L-monomethyl-L-arginine (L-NNMA), L-NG-methylarginine (L-NMA), Nw-nitro-L-arginine (L-NA), and aminoguanidine, and the substance that removes the produced nitric oxide may be any one or more of non-heme iron-containing peptides or proteins, porphyrins, metalloporphyrins, dithiocarbamates, dimercaptosuccinic acid, phenanthroline, desferrioxamine, pyridoxal isonicotinoyl hydrazone (PIH), 1,2-dimethyl-3-hydroxypyrid-4-one (L1), and [+] 1,2-bis(3,5-dioxopiperazine-1-yl)propane (ICRF-187). Specifically, the nitric oxide inhibitor may be a substance that inhibits nitric oxide production, and more specifically, may be NG-nitro-L-arginine methyl ester (L-NAME).

In one embodiment of the present invention, the administration of the nitric oxide (NO) inhibitor may be by any one route selected from among oral, parenteral, intravascular, intradermal, transdermal, intramuscular, intraperitoneal, intravenous, subcutaneous, and intranasal routes, and specifically, the nitric oxide inhibitor may be administered intraperitoneally.

The nitric oxide inhibitor may be administered one or more times, and the nitric oxide inhibitor may be administered in an amount of 50 to 500 mg/kg, specifically 100 to 400 mg/kg.

In one embodiment of the present invention, the administration of the nitric oxide (NO) inhibitor may be performed before or after the ligation of the common carotid artery. Specifically, the administration of the nitric oxide (NO) inhibitor may be performed immediately before or after the ligation of the common carotid artery. That is, in the present invention, the administration of the nitric oxide (NO) inhibitor and the ligation of the common carotid artery may be performed in any order.

In another embodiment of the present invention, the cerebrovascular disease may be at least one selected from the group consisting of palsy, stroke, cerebral hemorrhage, cerebral infarction, Alzheimer's disease, and vascular dementia, without being limited thereto. Specifically, the cerebrovascular disease may be cerebral infarction, more specifically, acute cerebral infarction.

The animal model refers to a subject in which cerebrovascular disease may occur. More specifically, the animal model is a non-human mammal, and examples of the non-human mammal include, but are not limited to, mice, rats, dogs, cats, horses, cows, sheep, goats, pigs, and rabbits.

Through the method for producing animal models of cerebrovascular disease according to the present invention, it is possible to simply, quickly and stably provide animal models in which large cerebral infarction that occupies more than 600 of the cerebral hemisphere occurs, or medium-sized cerebral infarction that occupies 30 to 600 of the cerebral hemisphere occurs, small cerebral infarction that occupies less than 30% of the cerebral hemisphere occurs, or no cerebral infarction occurs. This can overcome the conventional complexity and low-yield problem, and the production method of the present invention is capable of producing animal models of cerebrovascular disease with high yield by a simple method.

Another aspect of the present invention provides animal models of cerebrovascular disease produced by the above-described production method.

The animal models of cerebrovascular disease according to the present invention are animal models in which cerebrovascular disease has been induced through the above-described production method, and include animal models in which large cerebral infarction that occupies more than 600 of the cerebral hemisphere occurs, or medium-sized cerebral infarction that occupies 30 to 600 of the cerebral hemisphere occurs, small cerebral infarction that occupies less than 30% of the cerebral hemisphere occurs, or no cerebral infarction occurs.

As described above, the animal models of cerebrovascular disease according to the present invention may be produced by a simpler method than a conventional method for producing animal models of cerebrovascular disease, and thus may be produced with high yield and may be used to verify the effectiveness and safety of substances for the diagnosis, prevention or treatment of cerebrovascular disease or methods for treatment of cerebrovascular disease.

In addition, although the animal models of the present invention are those produced by the same method, the level of cerebrovascular disease may be different for each individual, and the animal models of the present invention may provide the possibility of identifying and studying genes that cause such differences in the level of cerebrovascular disease. Therefore, as in the conventional art, even when cerebrovascular disease does not occur in the animal model, the animal model is not considered failure, but may be used to identify genes that cause differences in the level of cerebrovascular disease.

In addition, in the process of confirming the onset of cerebrovascular disease in the animal model of cerebrovascular disease according to the present invention, it is possible to simply and accurately determine the level of cerebrovascular disease in a non-invasive manner using laser speckle flowmetry without sacrificing the animal model for tissue staining or using expensive brain imaging equipment such as MRI.

In another embodiment of the present invention, the cerebrovascular disease may be at least one selected from the group consisting of palsy, stroke, cerebral hemorrhage, cerebral infarction, Alzheimer's disease, and vascular dementia, without being limited thereto. Specifically, the cerebrovascular disease may be cerebral infarction, more specifically, acute cerebral infarction.

The animal model refers to a subject in which cerebrovascular disease may occur. More specifically, the animal model is a non-human mammal, and examples of the non-human mammal include, but are not limited to, mice, rats, dogs, cats, horses, cows, sheep, goats, pigs, and rabbits.

Still another aspect of the present invention provides a method for producing animal models having uniform susceptibility to the onset of cerebrovascular disease, the method comprising a step of mating animal models having similar susceptibilities to the onset of cerebrovascular disease among the above-described animal models of cerebrovascular disease.

The "susceptibility" to the onset of cerebrovascular disease refers to a property that causes a difference in the level of cerebrovascular disease which occurs when treated by the above-described method for producing animal models of cerebrovascular disease. Specifically, it refers to resistance.

The animal models having "similar" susceptibilities to the onset of cerebrovascular disease refer to models that exhibit similar levels of cerebrovascular disease when treated by the above-described method for producing animal models of cerebrovascular disease. More specifically, when the presence or absence and size of cerebral infarction are predicted based on laser speckle flowmetry data, it is possible to determine whether or not cerebrovascular disease has occurred or similar susceptibilities to the onset of cerebrovascular disease, based on the criteria for distinguishing cases where large cerebral infarction that occupies more than 600 of the cerebral hemisphere has occurred, or medium-sized cerebral infarction that occupies 30 to 600 of the cerebral hemisphere has occurred, small cerebral infarction that occupies less than 30% of the cerebral hemisphere has occurred, or no cerebral infarction has occurred.

"Mating" of animal models having similar susceptibilities to the onset of cerebrovascular disease among the animal models of cerebrovascular disease may be performed by a known method. In addition, the animal models of cerebrovascular disease according to the present invention are in a state in which the common carotid artery is ligated, and these animal models may be mated after one week of stabilization in a state in which the ligation is temporarily released.

The present invention has an effect of providing a large amount of offspring animals having uniform susceptibility to the onset of cerebrovascular disease by mating animal models having similar susceptibilities to the onset of cerebrovascular disease among the animal models of cerebrovascular disease.

Yet another aspect of the present invention provides animal models having uniform susceptibility to the onset of cerebrovascular disease, produced by the above production method.

The animal models having uniform susceptibilities to the onset of cerebrovascular disease are those born by mating of animal models having similar susceptibilities to the onset of cerebrovascular disease. Since these animal models are born by mating of parent animal models having similar susceptibilities to the onset of cerebrovascular disease, they retain factors (e.g., genes) that determine susceptibility to the onset of cerebrovascular disease, and thus may have uniform susceptibility to the onset of cerebrovascular disease.

### Mode for Invention

Hereinafter, one or more specific embodiments will be described in more detail with reference to examples. However, these examples are intended to illustrate one or more specific embodiments, and the scope of the present invention is not limited to these examples.

### Comparative Example 1: Conventional method (Longa) for producing animal model of cerebrovascular disease

After incising the skin of the neck, each portion of the right carotid artery is dissected cleanly. The common carotid artery (CCA) is ligated, the pterygopharyngeal artery (PPA) is ligated, and then the external carotid artery (ECA) is partially ligated (= partial knot tying). While the distal ECA is held with a clip for acetabular surgery, an opening is made by partially incising the proximal ECA, and a nylon suture is inserted therein. Then, the clip is released, and the suture is advanced toward the internal carotid artery (ICA) and then immediately pushed in, and at the same time, the partial knot is made similar to a full knot to minimize bleeding. Then, while the nylon suture is gradually pushed in, the pushing is stopped when blood flow in the middle cerebral artery is observed to decrease to 20-30% compared to baseline on laser Doppler flowmetry, and the knot is tightened to immobilize the suture positioned to block the initiation site of the middle cerebral artery (FIG. 1).

### Example 1: Production of animal models of cerebrovascular disease according to the present invention

12-week-old mice (C57BL/6) fasted for one day (fed only drinking water) were weighed and subjected to inhalation anesthesia with 2% isoflurane. In this state, while the mice were maintained at a body temperature of 36.5 ± 0.5°C using a homeothermic blanket (Panlab), cerebral blood flow (CBF) started to be monitored using laser speckle flowmetry. In order to induce mild cerebral ischemia in the right hemisphere, the skin of the neck was incised and then the common carotid artery (CCA) was ligated under a surgical microscope (Leica, EZ4, 8X). As a next step, a nitric oxide synthesis inhibitor (N omega-nitro-L-arginine methyl ester hydrochloride (L-NAME)) was intraperitoneally administered once in an amount of 400 mg/kg. The skin of the neck was sutured and anesthesia was discontinued.

Unlike a conventional complicated method of inducing cerebral infarction, the above-described method for producing animal models according to the present invention employs a simple method of ligating only CCA (arrow) and intraperitoneally injecting a nitric oxide inhibitor, and does not require additional procedures such as lowering blood pressure or blocking oxygen supply after CCA ligation, unlike another conventional method of inducing cerebral infarction, indicating that the method of the present invention is capable of producing animal models of cerebrovascular disease in a simple and efficient manner.

### Experimental Example 1: Confirmation of occurrence of cerebrovascular disease

The present inventor examined whether cerebrovascular disease was induced through the method for producing animal models of cerebrovascular disease according to the present invention and whether cerebrovascular disease could be confirmed noninvasively through laser speckle flowmetry.

Specifically, the animal models of cerebrovascular disease produced in Example 1 were subjected to inhalation anesthesia with 2% isoflurane, and in this state, CBF images were acquired using laser speckle flowmetry before induction of cerebrovascular disease and 5 minutes and 24 hours after induction of cerebrovascular disease. In addition, after sacrificing the animals, the brain tissues were harvested, sectioned to 2 mm thickness, and then stained with a 2% 2,3,5-triphenyl tetrazolium chloride (TTC) solution, and the onset of cerebral infarction was checked, and laser speckle flowmetry results and cerebral infarction size were compared.

As a result, as shown in FIG. 2, it could be confirmed through the CBF images of laser speckle flowmetry that cerebrovascular disease (cerebral infarction) was induced in the right hemisphere through the production method of the present invention, which was consistent with the results of TTC solution staining. Specifically, in FIG. 2A, in the result of laser speckle flowmetry at 24 hours after induction of cerebrovascular disease, reduction in cerebral blood flow was extensively severe (arrow in laser speckle flowmetry; right hemisphere shown in dark blue color), and in the TTC staining image obtained after sacrificing the animal, large cerebral infarction was observed (arrow in TTC). In FIG. 2B, in the result of laser speckle flowmetry at 24 hours after induction of cerebrovascular disease, reduction in cerebral blood flow was not severe (arrow in laser speckle flowmetry arrow; some brain areas shown in light blue), and in the TTC staining image obtained after sacrificing the animal, small cerebral infarction was observed (arrow in TTC). In FIG. 2C, in the result of laser speckle flowmetry at 24 hours after induction of cerebrovascular disease, reduction in cerebral blood flow was very weak (arrow in laser speckle flowmetry; some light green brain areas), and in the TTC staining image obtained after sacrificing the animals, cerebral infarction was not observed.

This suggests that the method for producing animal models of cerebrovascular disease according to the present invention is capable of producing animal models of cerebrovascular disease, and that the onset of cerebrovascular disease in the animal models can be determined non-invasively through laser speckle flowmetry.

### Experimental Example 2: Verification of applicability as animal models of cerebrovascular disease

Whether the animal models of cerebrovascular disease produced through the production method of the present invention can be used as animal models of cerebrovascular disease was examined by applying them to drug screening.

Specifically, induction of cerebrovascular disease (cerebral infarction) in mice, to which cilostazol (50 mg/kg) known to be used to prevent cerebral infarction and have a cerebral vasodilator effect was orally administered twice a day for 1 week, was attempted by applying the production method of the present invention, and at 1 week after administration, the size of cerebral infarction was compared with the size of cerebral infarction induced in mice to which saline was administered for the same period (n=11 per group) .

As a result, as shown in FIG. 3, it can be confirmed that the size of cerebral infarction in the mice, to which cilostazol was administered, decreased compared to that in the saline-treated group (saline, control group) (39.0 ± 47.0 mm³ vs. 4.7 ± 6.0 mm³, p < 0.01 (t-test)). This suggests that the animal models of cerebrovascular disease according to the present invention are suitable for use in cerebrovascular disease research.

### Experimental Example 3: Identification of offspring animal models produced by mating of animal models of cerebrovascular disease

The present inventor examined whether, when cerebrovascular disease models were produced by the production method of the present invention, cerebrovascular disease occurred in offspring born by mating of animal models having similar levels of cerebrovascular disease among the animal models of cerebrovascular disease produced by the production method of the present invention, at a level similar to that in the parent animals, and whether it is possible through the animal models of the present invention to predict the presence of cerebrovascular disease-resistance genes and produce offspring animals having uniform level of cerebrovascular disease.

Based on the laser speckle flowmetry data of the animal models of Example 1, the presence or absence and size of cerebral infarction were predicted, and animal models showing similar prediction results were selected and grouped (selected and grouped into cases where large cerebral infarction that occupies more than 600 of the cerebral hemisphere occurred, or medium-sized cerebral infarction that occupies 30 to 600 of the cerebral hemisphere occurred, small cerebral infarction that occupies less than 30% of the cerebral hemisphere occurred, or no cerebral infarction occurred). Next, the animals were stabilized for 1 week in a state where ligation of the common carotid artery was released, it was confirmed whether mice born by mating the animals of the same group had a cerebral infarction size similar to that in their parents when the production method of the present invention was applied thereto (n = 10/group).

As a result, as shown in FIG. 4, it can be confirmed that, in offspring animals obtained by mating parent animals in which similar levels of cerebral infraction are induced through the production method of the present invention, cerebral infraction was also induced at a level similar to that in the parent animals when the production method of the present invention was applied to the offspring animals. This suggests that it is possible to predict the presence of genes exhibiting resistance to cerebrovascular disease, and to obtain offspring animals capable of exhibiting a uniform level of cerebrovascular disease by mating animal models having similar levels of cerebrovascular disease.

So far, the present invention has been described with reference to the preferred embodiments thereof. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A method for producing animal models of cerebrovascular disease, the method comprising ligation of a common carotid artery (CCA) and administration of a nitric oxide (NO) inhibitor.

2. The method of claim 1, wherein the ligation of the common carotid artery (CCA) is permanent ligation.

3. The method of claim 1, wherein the nitric oxide synthase (NOS) inhibitor is any one or more of NG-nitro-L-arginine methyl ester (L-NAME), NG-monoethyl-L-arginine (L-MMA), N-iminoethyl-L-ornithine (L-NIO), L-monomethyl-L-arginine (L-NNMA), L-NG-methylarginine (L-NMA), Nw-nitro-L-arginine (L-NA), and aminoguanidine.

4. The method of claim 1, wherein the administration of the nitric oxide synthase (NOS) inhibitor is by any one route selected from among oral, parenteral, intravascular, intradermal, transdermal, intramuscular, intraperitoneal, intravenous, subcutaneous, and intranasal routes.

5. The method of claim 1, wherein the administration of the nitric oxide synthase (NOS) inhibitor is performed before or after the ligation of the common carotid artery.

6. The method of claim 1, wherein the cerebrovascular disease is at least one selected from the group consisting of palsy, stroke, cerebral hemorrhage, cerebral infarction, Alzheimer's disease, and vascular dementia.

7. Animal models of cerebrovascular disease produced by the method of claim 1.

8. The animal models of claim 7, wherein the cerebrovascular disease is acute cerebrovascular disease.

9. A method for producing offspring animals having uniform susceptibility to onset of cerebrovascular disease, the method comprising a step of mating animal models having similar susceptibilities to onset of cerebrovascular disease among the animal models of cerebrovascular disease of claim 7.

10. Offspring animas having uniform susceptibility to onset of cerebrovascular disease, produced by the method of claim 9.
